# EUROPÄISCHE PATENTSCHRIFT

(11) **EP 0 567 458 B1**
(45) Veröffentlichungstag und Bekanntmachung des Hinweises auf die Patenterteilung: **28.08.1996**
(21) Anmeldenummer: 91919137.9
(22) Anmeldetag: 14.11.1991
(51) Int. Cl.: A61B 5/14

(54) **VORRICHTUNG ZUR ARTERIELLEN BLUTENTNAHME**
DEVICE FOR WITHDRAWING ARTERIAL BLOOD
DISPOSITIF DE PRELEVEMENT SANGUIN A PARTIR D'UNE ARTERE

(43) Veröffentlichungstag der Anmeldung: 03.11.1993
(73) Patentinhaber: AVL Medical Instruments AG, CH-8207 Schaffhausen (CH)
(72) Erfinder: SCHWARZ, Manfred, D-8084 Bachern (DE)
(74) Vertreter: Blum, Rudolf Emil Ernst
(86) Internationale Anmeldenummer: CH9100234
(87) Internationale Veröffentlichungsnummer: WO9309714

(56) Entgegenhaltungen:
- DE-A- 2 836 780
- DE-A- 4 015 468
- DE-U- 8 902 045

## Beschreibung

Die Erfindung bezieht sich auf eine Vorrichtung zur arteriellen Blutentnahme mit einem Schutzrohr mit Befestigungsteil, das einen konischen Sitz zur Aufnahme einer Kanüle aufweist und zwei in dem Schutzrohr gehaltenen Kapillaren, von denen die erste mit der Kanüle und die zweite über ein Umlenkteil, in dem die beiden Kapillaren in einem Sitz gehalten sind mit der ersten Kanüle in Verbindung steht, wobei die dem Umlenkteil abgewandten Enden der Kapillaren im Befestigungsteil gehalten sind und das Schutzrohr und das Befestigungsteil für die Kanülle einstückig ausgebildet sind.

Eine bekannte Blutentnahmevorrichtung dieser Art ist aus der DE 28 36 780 C2 bekannt.

Bei einer anderen bekannten, unter dem Handelsnamen "MIKRO-PUNKTIONS-KIT" (Prospekt der Firma AVL Medical Instruments AG, Schaffhausen, CH) erhältlichen Vorrichtung dieser Art sind Schutzrohr und Befestigungsteil getrennt voneinander ausgebildet, wobei das Befestigungsteil nach Art einer Kappe auf das Schutzrohr aufsteckbar ist. Vorrichtungen dieser Art arbeiten ohne Zuhilfenahme eines Saugkolbens, wobei das Blut aufgrund des Blutdruckes in die Kanüle und von dort in die Kapillaren einströmt. Zur Übertragung des entnommenen Blutes in ein Blutanalysegerät wird das als Kappe ausgebildete Befestigungsteil von dem Schutzrohr abgezogen, worauf die Kapillaren aus ihrem Sitz im Umlenkteil herausgezogen werden können.

Wegen einer leichten und sicheren Montage sowie auch Demontage des Befestigungsteiles wurde dieses bei der bekannten Vorrichtung aus einem weichen Kunststoff, vorzugsweise Polyäthylen hergestellt. Dieser Werkstoff ist jedoch nur durchscheinend, also nicht klar durchsichtig. Somit ist bei der bekannten Vorrichtung keine einwandfreie Kontrolle des letzten Füllbereiches der zweiten Kapillare möglich. Ebenso ist infolge der Weichheit des Werkstoffes keine feste Verbindung zwischen Kanüle und konischem Sitz des Befestigungsteiles erzielbar. Insofern besteht die Gefahr eines unbeabsichtigten Lösens der Kanüle vom Befestigungsteil, wodurch der Behandelnde mit dem Blut des Patienten in Berührung kommen kann. Die lagerichtige Montage in bezug auf die beiden ungleich langen Kapillaren, sowie die Forderung einer handlichen, geringen Baugröße, führten zum ovalen Querschnitt der bekannten Vorrichtung. Dieser Querschnitt ist jedoch hinsichtlich Dichtheit bei der Montage des Umlenkteiles auf dem Schutzrohr schwer beherrschbar.

Bei der eingangs erwähnten bekannten Vorrichtung nach der DE 28 36 780 C2 ist zwar der Nachteil des losen Befestigungsteiles, welches als Kappe ausgebildet ist, beseitigt, weil das Befestigungsteil und das Schutzrohr einteilig ausgeführt sind, doch verbleibt der Nachteil des ovalen Querschnitts des Schutzrohres, weil die beiden Kapillaren nach wie vor parallel nebeneinander angeordnet sind.

Neuere Blutanalysegeräte benötigen aufgrund erweiterter Analysen ein größeres Blutvolumen. Dies ist jedoch ohne entscheidende Änderung der bekannten Vorrichtung nur über die Vergrößerung des Innendurchmessers der Kapillaren erzielbar. Dieser Vergrößerung sind jedoch Grenzen gesetzt. Zum einen erfordert die Montage und spätere Entnahme der Kapillaren aus der Vorrichtung eine gewisse Stabilität, d. h. Sicherheit vor Bruch, und somit eine gewisse Mindestwandstärke der aus Glas bestehenden Kapillaren. Zum anderen ist der Durchmesser nicht beliebig erweiterbar, da zum Erhalt einer einwandfreien Blutanalyse das Blut vor der vorzeitigen Gerinnung bewahrt werden muß. Aufgrund dessen werden die Kapillaren an der Oberfläche der Innenseite heparinisiert, d. h. mit einem gerinnungshemmenden Chemikal versehen. Die Benetzungsmöglichkeit, d. h. die Wirkung des Heparins auf den Blutstrom in der Kapillare nimmt jedoch mit zunehmenden Abstand der beaufschlagten Wände ab. Somit kann es im Inneren des Blutstromes zu parziellen Gerinnungen kommen, welche die Analyse negativ beeinflussen.

Aufgabe der Erfindung ist es, eine Vorrichtung der eingangs erwähnten Art so auszugestalten, daß bei einem vergrößerten Blutaufnahmevolumen eine sicherere Handhabung und eine erleichterte Montage möglich ist.

Diese Aufgabe wird ausgehend von einer Vorrichtung nach dem Oberbegriff des Anspruches 1 erfindungsgemäß dadurch gelöst, daß die erste Kapillare konzentrisch in der zweiten Kapillare angeordnet ist und beidseitig über die zweite Kapillare hervorsteht.

Durch die Anordnung der ersten Kapillare innerhalb der zweiten Kapillare entsteht innerhalb der zweiten Kapillare trotz geringer Schichtdicke des Blutes, welches sich zwischen der ersten und zweiten Kapillare ansammelt, ein großes Volumen, ohne daß die Gesamtvorrichtung vergrössert werden müßte. Außerdem ist die Herstellung des Schutzrohres und des Befestigungsteiles hinsichtlich der Ausbildung der jeweiligen Sitze für die Kapillaren durch diese konzentrisch ineinander gesteckte Anordnung der Kapillaren vereinfacht.

Eine besonders vorteilhafte Ausgestaltung der Erfindung ergibt sich dadurch, daß das Befestigungsteil einen den konischen Sitz für die Kanüle umgebenden, in Richtung auf das verjüngte Ende des konischen Sitzes offenen, topfartigen Kragen aufweist, der mit dem Befestigungsteil einstückig ausgeführt ist und einen Bajonettverschluß für die Kanüle aufweist. Dieser Bajonettverschluß ermöglicht ein sicheres Festhalten der Kanüle auf dem konischen Sitz.

Eine vorteilhafte Ausgestaltung des Bajonettverschlusses besteht darin, daß der Kragen eine den beiden flanschartigen Ansätzen der handelsüblichen Kanüle angepaßte, im wesentlichen ellipsenförmige Einführöffnung und zwei in Richtung der kleinen Achse der Ellipse einander gegenüberliegende Durchbrechungen aufweist, in die die beiden Ansätze der Kanüle in der festgelegten Stellung eingreifen. In Verbindung mit einer weiteren Ausgestaltung, wonach die der offenen Seite des Kragens zugewandten Begrenzungsflächen der Durchbrechungen schräg zur Längsachse des Schutzrohres verlaufen, wird nach Art eines Gewindes ein Festziehen der Kanüle auf den Sitz ermöglicht, so daß eine gute Abdichtung zwischen Kanüle und Sitz erzielbar ist. Außerdem ist ein zufälliges Lösen der Kanüle von ihrem Sitz durch den formschlüssigen Eingriff der Ansätze in die Durchbrechungen verhindert, so daß die Handhabung der Vorrichtung wesentlich sicherer ist als bei der bekannten Vorrichtung, bei der sich die Kanüle von dem Sitz wegen Fehlens einer formschlüssigen Verbindung lösen konnte.

Die Ausbildung eines Bajonettverschlusses an einer Blutentnahmevorrichtung zur Befestigung der Kanüle ist zwar grundsätzlich aus der DE 30 49 503 A1 bekannt, doch ist dort die Kanüle an einer zylindrischen Hülse gehalten, die auf einen zylindrischen Ansatz am Schutzrohr aufschiebbar ist und einen Längsschlitz mit einem unter einem stumpfen Winkel abknickenden Endschlitz aufweist, in den ein stiftförmiger Haltenocken am zylindrischen Ansatz des Schutzrohres eingreift. Diese Verbindung zwischen Kanüle und Schutzrohr ist wegen Fehlens des konischen Sitzes und der Ausbildung des Bajonettverschlusses in Form von Durchbrechungen in einem den konischen Sitz umgebenden Kragen mit Schrägflächen zum festen Aufdrücken der Kanüle auf den konischen Sitz nicht so sicher und fest, daß man ohne zusätzliche Abdichtung auskommen würde. Vielmehr ist bei der bekannten Ausgestaltung eine zusätzliche Abdichtung der Kanüle notwendig.

Wenn in weiterer Ausgestaltung der Erfindung das Schutzrohr einen kreisförmigen Querschnitt aufweist, so ist hierdurch nicht nur eine besonders gedrängte Bauart möglich, sondern auch die Ausbildung der jeweiligen Sitzflächen für die jenigen Enden der Kapilaren erleichtert, die der Känüle zugewandt sind. Dieser kreisförmige Querschnitt wird durch die konzentrische Anordnung der Kapillaren ermöglicht.

Es erweist sich als besonders vorteilhaft, wenn in weiterer Ausgestaltung der Erfindung das Umlenkteil als Stopfen aus einem weichen Kunststoff ausgebildet und in das Schutzrohr lösbar einsteckbar ist und zwei konzentrische sacklochartige Bohrungen als jeweiligen Sitz für die Aufnahme der Kapillaren aufweist, deren Bohrungsgrund als stirnseitiges Widerlager für die Kapillaren dient und daß die innere Bohrung zumindest einen Querschlitz aufweist, der sich in radialer Richtung bis zur Innenwand der äußeren Kapillare und in Längsrichtung ausgehend vom Widerlager für die äußere Kapillare bis in das Widerlager für die innere Kapillare hinein erstreckt. Diese Ausgestaltung ermöglicht ein einfaches Entnehmen der Kapillaren aus dem Schutzrohr, so daß die Bruchgefahr bei aus Glas bestehenden Kapillaren verringert und somit eine sichere Handhabung der Vorrichtung gewährleistet ist. Da das als Stopfen ausgebildete Umlenkteil lösbar in das Schutzrohr eingesteckt ist und da dieser Stopfen aus einem weichen Kunststoff besteht, ergibt sich der wesentliche Vorteil, daß beim festen Aufstecken der Kanüle auf den Sitz am Befestigungsteil keine Bruchgefahr für die erste Kapillare besteht, da einerseits der Stopfen nachgiebig ist und andererseits gegenüber dem Schutzrohr ausweichen kann, wenn aufgrund von Fertigungstoleranzen die erste Kapillare zu weit aus dem Befestigungsteil hervorsteht, so daß die Kanüle gegen die Kapillare drücken kann. Bei der bekannten Vorrichtung konnte es dabei zu einem Bruch dieser ersten Kapillare kommen. Durch die Anordnung zumindest eines Querschlitzes werden zwei Überleitungsmöglichkeiten von der inneren Kapillare zur äußeren Kapillare geschaffen. Bevorzugt werden zwei sich kreuzende Querschlitze vorgesehen, so daß vier Überleitungsmöglichkeiten bestehen.

Wenn in weiterer vorteilhafter Ausgestaltung das Umlenkteil einen von der Kreisform abweichenden, vorzugsweise viereckigen, über das Schutzrohr radial hervorstehenden Flansch aufweist, so wird ein Wegrollen der im Querschnitt kreisrunden Vorrichtung bei der Ablage auf einem Tisch vermieden.

Die Erfindung wird nachstehend anhand eines bevorzugten Ausführungsbeispieles näher erläutert. In der Zeichnung zeigen:
- Fig. 1 :: einen Schnitt durch eine bekannte Vorrichtung;
- Fig. 2 :: eine Ansicht einer Vorrichtung nach der Erfindung;
- Fig. 3 :: einen Längsschnitt durch die Vorrichtung nach Fig.2;
- Fig. 4 :: einen Schnitt nach der Linie IV - IV in Fig. 2;
- Fig. 5 :: einen Schnitt nach der Linie V - V in Fig. 3.

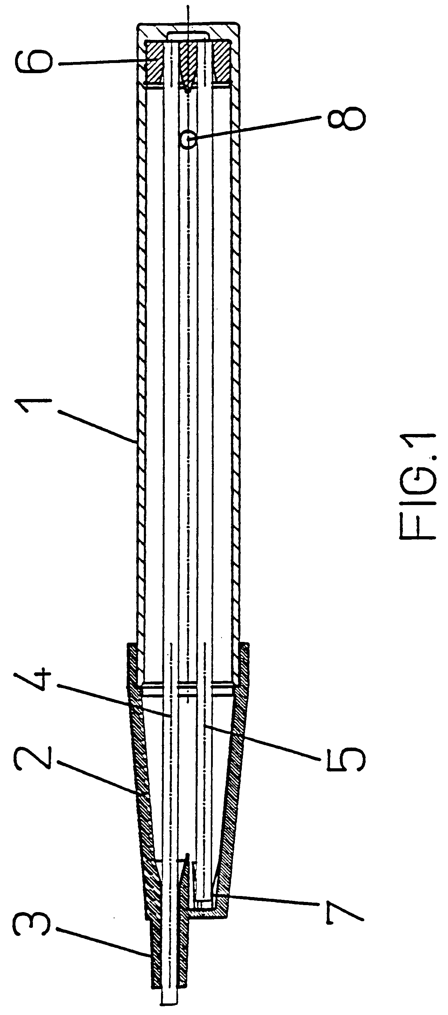

Die bekannte Vorrichtung nach Fig. 1 umfaßt ein Schutzrohr 1, ein Befestigungsteil 2 mit daran angeformten konischen Sitz 3 für eine nicht dargestellte Kanüle, eine erste Kapillare 4, eine zweite Kapillare 5 und einen Umlenkteil 6. Die erste Kapillare 4 sitzt mit ihrem einen Ende im Umlenkteil. Mit ihrem anderen Ende ist die erste Kapillare im Befestigungsteil 2 gehalten und ragt mit diesem Ende geringfügig über den konischen Sitz 3 hinaus. Die zweite Kapillare 5 sitzt ebenfalls im Umlenkteil 6, welches eine Verbindung zwischen der ersten und der zweiten Kapillare 4 und 5 herstellt. Das dem Umlenkteil 6 gegenüberliegende Ende der zweiten Kapillare 5 ist in einem Sitz 7 des Befestigungsteiles 2 gehalten, welches auf das Schutzrohr 1 aufgesteckt ist. Das Schutzrohr 1 hat im wesentlichen einen ovalen Querschnitt und besteht aus einem durchsichtigen Kunststoff während das Befestigungsteil 2 aus einem weichen, nur durchscheinenden Kunststoff hergestellt ist, das an seinem Ende, mit dem es auf das Schutzrohr 1 aufgeschoben ist, dem Querschnitt des Schutzrohres angepaßt ist. In Richtung auf den konischen Sitz 3 läuft das Befestigungsteil im wesentlichen konisch zu. Zwei Entlüftungsbohrungen 8 sind vorgesehen, um die beim Auffüllen der Kapillaren entweichende Luft aus dem Schutzrohr austreten zu lassen. Die Kapillaren sind aus Glas hergestellt.

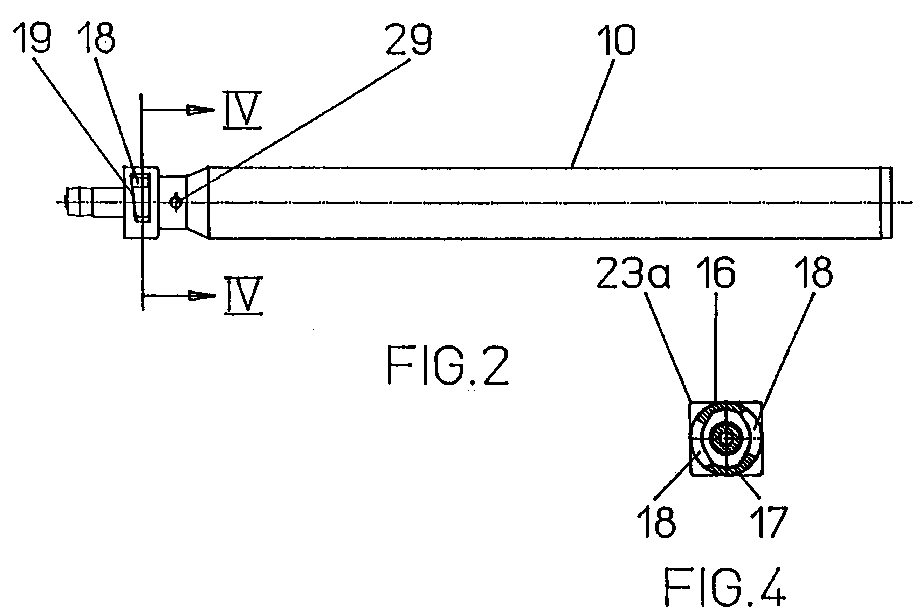

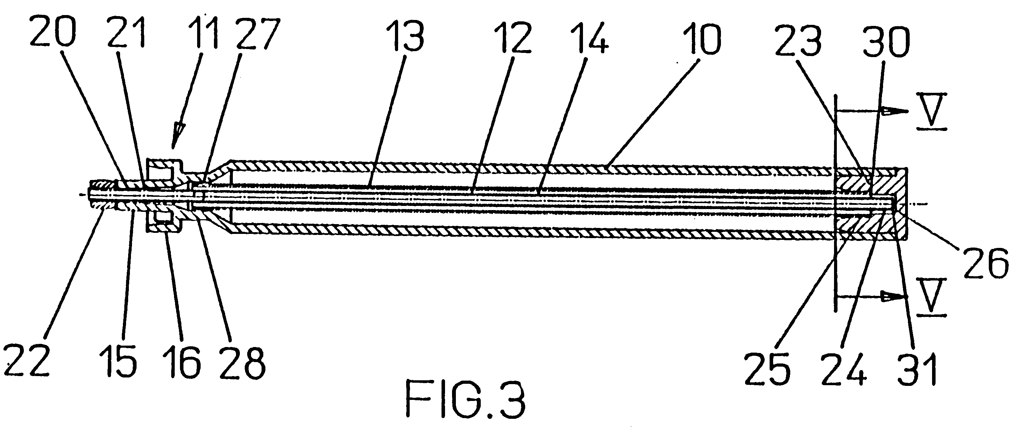

Die Figuren 2 bis 5 zeigen die erfindungsgemäße Vorrichtung. Diese besteht aus einem im Querschnitt kreisrunden Schutzrohr 10, an welchem ein insgesamt mit 11 bezeichnetes Befestigungsteil einstückig angeformt ist. Das Schutzrohr 10 und Befestigungsteil 11 bestehen aus einem durchsichtigen, verhältnismäßig harten thermoplastischen Kunststoff.

Konzentrisch zum Schutzrohr 10 sind zwei Kapillaren 12 und 13 in diesem angeordnet, wobei die erste Kapillare 12 konzentrisch innerhalb der zweiten Kapillare 13 eingesetzt ist und zwischen beiden Kapillaren ein Ringraum 14 verbleibt.

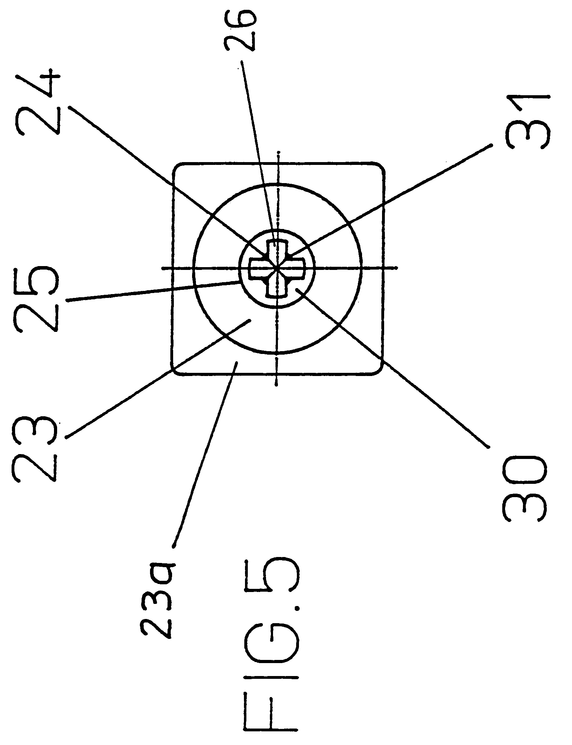

Das Befestigungsteil 11 weist einen konischen Sitz 15 auf einem tüllenartigen Ansatz 20 und einen diesen am dickeren Ende umgebenden topfartigen Kragen 16 auf. Der topfartige Kragen 16 begrenzt, wie aus Fig. 4 ersichtlich, eine im wesentlichen ellipsenförmige Einführöffnung 17 für eine nicht dargestellte handelsübliche Kanüle, die an ihrem Befestigungsende zwei einander gegenüberliegende flanschartige Ansätze aufweist und in diesem Bereich der Einführöffnung 17 angepaßt ist. In Richtung der kleinen Achse der ellipsenförmigen Öffnung 17 weist der Kragen 16 zwei Durchbrüche 18 auf, deren dem Schutzrohr 10 abgewandte Begrenzungsflächen 19 schräg zur Längsachse des Schutzrohres verlaufen, so daß die in die Einführöffnung 17 eingeführten flanschartigen Ansätze der Kanüle nach Verdrehen derselben bis zu ihrem Einrasten in die Durchbrechungen 18, an dieser schrägen Fläche 19 anliegen, wodurch bei einem weiteren Verdrehen der Kanüle diese fest auf den konischen Sitz 17 nach Art eines Gewindes gezogen wird. Der Kragen 16 bildet mit seinen Durchbrechungen 18 einen Bajonettverschluß, der ein formschlüssiges Festlegen der Kanüle am Befestigungsteil 11 gestattet.

Die erste Kapillare 12 ist einerseits innerhalb des an seiner Außenseite den konischen Sitz 15 aufweisenden tüllenartigen Ansatz 20 gehalten, der eine durchgehende Bohrung 21 aufweist, welche einen Sitz für die erste Kapillare 12 bildet. Die Kapillare 12 ragt über den Ansatz 20 nach außen hervor und kann von einer Dichtung 22 umgeben sein, falls besonders hohe Anforderungen an die Dichtheit gestellt werden.

Das dem Befestigungsteil 11 zugewandte Ende der zweiten Kapillare 13 ist in einem Sitz 27 des Befestigungsteiles 11 gehalten, der als durchgehende Bohrung in einem im Durchmesser gegenüber dem Schutzrohr 10 verkleinerten Übergangsstück 28 ausgebildet ist.

Die dem Befestigungsteil gegenüberliegenden Enden der Kapillaren 12 und 13 sind in einem als Umlenkteil dienenden Stopfen 23 eingesteckt, der zwei konzentrische sacklochartige Bohrungen 24 und 25 aufweist, wobei die innere Kapillare 12, die über die äußere Kapillare hinausragt, in der Bohrung 24 und die äußere Kapillare in der Bohrung 25 aufgenommen ist. Der jeweilige Bohrungsgrund 30 bzw. 31 der Bohrungen 24 und 25 dient als stirnseitiges Widerlager für die Kapillaren 12 und 13. Die innere, weiter in den Stopfen 23 hineinragende Bohrung 24 weist zwei kreuzweise angeordnete Querschlitze 26 auf, die sich in radialer Richtung bis zur Innenwand der äußeren Kapillare 13 und in Längsrichtung ausgehend vom Widerlager 30 bis in das Widerlager 31 hinein erstrecken, so daß sie vier Überleitungen bzw. Verbindungen zwischen der inneren Kapillare 12 und der äußeren Kapillare 13 schaffen. Das in der inneren Kapillare 12 strömende Blut wird über die Schlitze 26 in die äußere Kapillare geleitet. Da das Schutzrohr 10 und das Befestigungsteil 11 aus dem gleichen durchsichtigen Material bestehen, kann beim Abnehmen von Blut genau beobachtet werden, wie weit das Blut nach dem Überströmen aus der Kapillare 12 über die Schlitze 26 den Ringraum 14 füllt, so daß ein Austreten von Blut an dem den Sitz 27 zugeordneten Ende vermieden werden kann.

Der als Umlenkteil 23 dienende Stopfen besteht aus einem weichen Kunststoff, so daß er nachgeben kann, falls die erste Kapillare 12 zu weit aus dem Befestigungsteil herausragt und durch die aufgesetzte Kanüle nach hinten gedrückt wird. Zum Entnehmen der Kapillaren wird das Umlenkteil 23 aus dem Schutzrohr 10 herausgezogen, wodurch die beiden Kapillaren 12 und 13 aus dem Schutzrohr 10 mit herausgenommen werden können. Hierfür ist es erforderlich, daß die Kapillaren 12 und 13 stärker in dem Umlenkteil 23 als an ihren gegenüberliegenden Enden in ihren entsprechenden Sitzen gehalten sind, was aufgrund des weichen Kunststoffes ohne weiteres einstellbar ist. Die so entnommenen Kapillaren können dann in ein Blutanalysegerät eingelegt werden. Je nach Beschaffenheit des Blutanalysegerätes kann es aber auch möglich sein, daß die Kapillaren zusammen mit dem Schutzrohr in das Analysegeräte eingesetzt werden. Mit 29 sind zwei gegenüberliegende Entlüftungsbohrungen im Schutzrohr bezeichnet.

Das Umlenkteil 23 weist einen viereckigen Flansch 23a auf, der einerseits das Herausziehen dieses Umlenkteils aus dem Schutzrohr erleichtert, da er als Handhabe hierfür dient und andererseits ein Wegrollen auf einem Tisch behindert.

## Patentansprüche

1. Vorrichtung zur arteriellen Blutentnahme mit einem Schutzrohr (10) mit Befestigungsteil (11), das einen konischen Sitz (15) zur Aufnahme einer Kanüle aufweist und zwei in dem Schutzrohr (10) gehaltenen Kapillaren (12, 13) von denen die erste mit der Kanüle und die zweite über ein Umlenkteil (23), in dem die beiden Kapillaren (12, 13) in einem Sitz gehalten sind, mit der ersten Kapillare (12) in Verbindung steht, wobei die dem Umlenkteil (23) abgewandten Enden der Kapillaren (12, 13) im Befestigungsteil (11) gehalten sind und das Schutzrohr (10) und das Befestigungsteil (11) für die Kanüle einstückig ausgebildet sind, **dadurch gekennzeichnet,** daß die erste Kapillare (12) konzentrisch in der zweiten Kapillare (13) angeordnet ist und beidseitig über die zweite Kapillare (13) hervorsteht.

2. Vorrichtung nach Anspruch 1, **dadurch gekennzeichnet,** daß das Befestigungsteil (11) einen den konischen Sitz (15) für die Kanüle umgebenden, in Richtung auf das verjüngte Ende des konischen Sitzes offenen, topfartigen Kragen (16) aufweist, der mit dem Befestigungsteil (11) einstückig ausgeführt ist und einen Bajonettverschluß (18, 19) für die Kanüle aufweist.

3. Vorrichtung nach Anspruch 2, **dadurch gekennzeichnet,** daß der Kragen (16) eine den beiden flanschartigen Ansätzen der Kanüle angepaßte, im wesentlichen ellipsenförmige Einführöffnung (17) und zwei in Richtung der kleinen Achse der Ellipse einander gegenüberliegende Durchbrechungen (18) aufweist, in die die beiden Ansätze der Kanüle in der festgelegten Stellung eingreifen.

4. Vorrichtung nach Anspruch 3, **dadurch gekennzeichnet,** daß die der offenen Seite des Kragens (16) zugewandten Begrenzungsflächen (19) der Durchbrechungen (18) schräg zur Längsachse des Schutzrohres (10) verlaufen.

5. Vorrichtung nach einem der Ansprüche 1 bis 4, **dadurch gekennzeichnet,** daß das Schutzrohr (10) einen kreisförmigen Querschnitt aufweist.

6. Vorrichtung nach einem der Ansprüche 1 bis 5, **dadurch gekennzeichnet,** daß das Umlenkteil (23) als Stopfen aus einem weichen Kunststoff ausgebildet und in das Schutzrohr (10) lösbar einsteckbar ist und zwei konzentrische sacklochartige Bohrungen (24, 25) als jeweiligen Sitz für die Aufnahme der Kapillaren (12, 13) aufweist, deren Bohrungsgrund als stirnseitiges Widerlager (30, 31) für die Kapillaren (12, 13) dient und daß die innere Bohrung (24) zumindest einen Querschlitz (26) aufweist, der sich in radialer Richtung bis zur Innenwand der äußeren Kapillare (13) und in Längsrichtung ausgehend vom Widerlager (30) für die äußere Kapillare (13) bis in das Widerlager (31) für die innere Kapillare (12) hinein erstreckt.

7. Vorrichtung nach einem der Ansprüche 1 bis 6, **dadurch gekennzeichnet,** daß das Umlenkteil (23) einen von der Kreisform abweichenden, vorzugsweise viereckigen, über das Schutzrohr (10) radial hervorstehenden Flansch (23a) aufweist.

## Claims

1. Apparatus for withdrawing arterial blood, with a protective tube (10) with a mounting member (11), which includes a conical seat (15) for the receipt of a hollow needle, and two capillaries (12, 13) held in the protective tube (10) of which the first is in communication with the hollow needle and the second with the first capillary (12) via a deflecting member (23) which holds the two capillaries (12, 13) in a seat, whereby the ends of the capillaries (12, 13) facing away from the deflecting member (23) are held in the mounting member (11) and the protective tube (10) and the mounting member (11) for the hollow needle are made integrally, characterized in that the first capillary (12) is arranged concentrically in the second capillary (13) and projects at both ends beyond the second capillary (13).

2. Apparatus according to claim 1, characterized in that the mounting member (11) comprises a pot-like collar (16) surrounding the conical seat (15) for the hollow needle and open in the direction of the tapered end of the conical seat, which is made integrally with the mounting member (11) and includes a bayonet coupling (18, 19) for the hollow needle.

3. Apparatus according to claim 2, characterized in that the collar (16) comprises a substantially elliptically shaped insertion opening (17) made to suit the two flange-like projections of the hollow needle, and two openings (18) located opposite of each other in the direction of the short axis of the ellipse, into which the two projections of the hollow needle engage in the arrested position.

4. Apparatus according to claim 3, characterized in that the limiting surfaces (19) of the openings (18) facing the open side of the collar (16) extend obliquely relative to the longitudinal axis of the protective tube (10).

5. Apparatus according to one of the claims 1 to 4, characterized in that the protective tube (10) has a circular cross-section.

6. Apparatus according to one of the claims 1 to 5, characterized in that the deflecting member (23) is made as a plug of a soft plastic material and is releasably plugged into the protective tube (10) and includes two concentric blind hole-like bores (24, 25) as respective seat for each of the capillaries (12, 13), the bottom of the bores serving as frontal abutment (30, 31) for the capillaries (12, 13), and in that the inner bore (24) includes at least one transversal slot (26) which extends in radial direction up to the inner wall of the outer capillary (13) and in longitudinal direction from the abutment (30) for the outer capillary (13) up into the abutment (31) for the inner capillary (12).

7. Apparatus according to one of the claims 1 to 6, characterized in that the deflecting member (23) comprises a non-circular flange (23a) which is preferably square, and projects radially over the protective tube (10).

## Revendications

1. Dispositif de prélèvement sanguin à partir d'une artère, avec un tube protecteur (10) comportant une partie d'accouplement (11) présentant un siège conique (15) pour recevoir une canule, et avec deux capillaires (12,13) maintenues dans le tube protecteur (10) et dont la première communique avec la canule et la seconde communique, par l'intermédiaire d'un organe de déviation (23) maintenant les deux capillaires (12, 13) dans un siège, avec la première (12), les extrémités des capillaires (12, 13) éloignées de l'organe de déviation (23) étant maintenues dans la partie d'accouplement (11) faite d'une pièce avec le tube de protection (10), caractérisé en ce que la première capillaire (12) est disposée concentriquement dans la seconde (13) et dépasse cette dernière (13) aux deux extrémités.

2. Dispositif selon la revendication 1, caractérisé en ce que la partie d'accouplement (11) comporte un col (16) en forme de bol entourant le siège conique (15) destiné à la canule et ouvert en direction de la petite extrémité de ce siège, ce col faisant partie intégrale de la partie d'accouplement (11) et comportant un accouplement à baïonnette (18, 19) pour la canule.

3. Dispositif selon la revendication 2, caractérisé en ce que le col (16) comporte une embouchure (17) de forme essentiellement elliptique adaptée aux deux ailettes en forme de flasque de la canule, ainsi que deux ouvertures (18) se faisant face en direction du petit axe de l'ellipse, dans lesquelles viennent se loger les deux ailettes de la canule en position bloquée.

4. Dispositif selon la revendication 3, caractérisé en ce que les surfaces (19) limitant les ouvertures (18) en direction du côté ouvert du col (16) sont inclinées par rapport à l'axe longitudinal du tube de protection (10).

5. Dispositif selon une des revendications 1 à 4, caractérisé en ce que le tube de protection (10) a une section circulaire.

6. Dispositif selon une des revendications 1 à 5, caractérisé en ce que l'organe de déviation (23) est constitué par un bouchon en matière plastique molle pouvant être enfoncé dans le tube protecteur (10) et retiré de celui-ci, et comportant deux trous borgnes concentriques (24, 25) formant sièges pour recevoir les capillaires (12, 13), le fond des trous servant d'appui frontal (30, 31) pour les capillaires (12, 13), et que le trou intérieur (24) présente au moins une fente transversale (26) qui s'étend radialement jusqu'à la paroi intérieure de la capillaire extérieure (13) et longitudinalement depuis l'appui frontal (30) pour la capillaire extérieure (13) jusqu'à l'appui frontal (31) pour la capillaire intérieure (12).

7. Dispositif selon une des revendications 1 à 6, caractérisé en ce que l'organe de déviation (23) comporte une flasque (23a) non-circulaire, de préférence carrée, débordant radialement du tube protecteur (10).
